# EUROPEAN PATENT SPECIFICATION

(11) **EP 3 397 969 B1**
(45) Date of publication and mention of the grant of the patent: **06.09.2023**
(21) Application number: 16826367.1
(22) Date of filing: 30.12.2016
(51) Int. Cl.: G01N 33/68

(54) **METHODS FOR MASS SPECTROMETRY-BASED STRUCTURE DETERMINATION OF BIOMACROMOLECULES**
VERFAHREN ZUR MASSENSPEKTROMETRISCHEN STRUKTURBESTIMMUNG VON BIOMOLEKÜLEN
PROCÉDÉ SPECTROMÉTRIQUE DE MASSE POUR DETERMINATION DE LA STRUCTURE DES BIOMOLÉCULES

(30) Priority: 30.12.2015 WO PCT/EP2015/203056
(43) Date of publication of application: 07.11.2018
(73) Proprietor: VITO NV, 2400 Mol (BE); Universiteit Antwerpen, 2000 Antwerpen (BE)
(72) Inventor: VALKENBORG, Dirk, 2400 Mol (BE); HOOYBERGHS, Jef, 2400 Mol (BE); LAUKENS, Kris, 2610 Wilrijk (BE)
(74) Representative: V.O.
(86) International application number: PCT/EP2016/082907
(87) International publication number: WO 2017/114943

(56) References cited:
- EP-A1- 2 081 025
- JP-A- 2006 294 014
- GEER L Y ET AL: "Open mass spectrometry search algorithm", INTERNET CITATION, 1 June 2004 (2004-06-01), XP002528323, Retrieved from the Internet: URL:http://arxiv.org/abs/q-bio/0406002 [retrieved on 2009-05-15]
- ARI M. FRANK ET AL: "Clustering Millions of Tandem Mass Spectra", JOURNAL OF PROTEOME RESEARCH., vol. 7, no. 1, 1 January 2008 (2008-01-01) , pages 113-122, XP055256428, US ISSN: 1535-3893, DOI: 10.1021/pr070361e
- THOMAS WILHELM ET AL: "Identification of Related Peptides through the Analysis of Fragment Ion Mass Shifts", JOURNAL OF PROTEOME RESEARCH., vol. 13, no. 9, 5 September 2014 (2014-09-05), pages 4002-4011, XP055256436, US ISSN: 1535-3893, DOI: 10.1021/pr500347e
- JOHN C. TRAN ET AL: "Mapping intact protein isoforms in discovery mode using top-down proteomics", NATURE, vol. 480, no. 7376, 30 October 2011 (2011-10-30), pages 254-258, XP055256392, United Kingdom ISSN: 0028-0836, DOI: 10.1038/nature10575 cited in the application

## Description

### FIELD OF THE INVENTION

The invention provides methods and tools for determining the structure of proteins in a sample using mass spectrometry.

### BACKGROUND

Large scale proteomics gained much attention once it became possible to acquire a large number of spectra (Michalski, A. et al., 2011, Mol. Cell. Proteomics MCP 10, M111.011015), and to identify a reasonable proportion of the resulting spectra in an automated fashion (Eng et al. (1994) J. Am. Soc. Mass Spectrom. 5, 976-989). Nevertheless, to this day between 33% to 50% of the acquired spectra remain unidentified. Essentially all proteomic work to date has been done at the peptide level by first enzymatically digesting the proteins to shorter peptides with more homogenous and tractable chemical characteristics. These are then introduced (usually following some form of physical/chemical separation) into a mass-spectrometer where spectra are acquired. The work of generating peptide spectral matches (PSMs) is then taken on by one of three styles of identification algorithms: (1) database searching tools compare the observed spectra to predicted spectra based on in-silico digestion of the target organism's proteome , (2) Tag-based de-novo techniques attempt to identify the peptide by direct interpretation of the spectrum followed by comparison to a database of predicted peptides and (3) the use of spectral libraries to identify the peptide by direct comparison of the query spectrum against a library of empirically derived reference spectra (usually referred to as a spectral library).

All of these approaches (including spectral libraries) rely heavily on the value of the query peptide's total mass (prior to tandem MS-fragmentation) typically referred to as the precursor mass. This is because the overall mass allows the algorithm to exclude a large proportion of the possible candidate peptides. The list of candidate peptides is itself kept to a minimum by the use of enzymes with sufficient specificity to produce only a relatively limited number of peptides per protein. Users typically ignore or severly limit the set of possible post translational modifications which the protein may have undergone (in-vivo) or the peptide itself may have acquired (in-vitro). The common thread to all peptide-centric approaches to proteomics is the requirement to: (1) know the proteome (sequence) of the target organism, (2) rely on a highly specific enzyme digestion process, and (3) filter candidate peptides by the precursor mass which yielded the observed fragment peaks. Furthermore, these techniques all assume that observed peptide spectra will be matched with predicted (in-silico derived) peptide spectra.

An alternative approach to mass-spec based proteomics is to avoid the digestion step and attempt to identify the protein by direct fragmentation of the entire protein in the mass-spectrometer (Tran, J. C. et al., 2011, Nature 480, 254-258). This presents, in principle, a very strong benefit in terms of characterization of the so-called proteoform (the overall combination of post translational modifications (PTMs) co-occuring on the protein - essentially its overall state), but the cost is a drastic limitation in the throughput of the technique as well as a strong increase in the complexity of the resulting spectra. This is because the approach is still in its infancy as evidenced by the relatively few published studies involving anything approaching high-throughput in the bottom-up realm. Nevertheless, computational techniques have been developed to match spectra generated from the entire protein (especially after a pre-processing step called de-convolution) to its putative source (see e.g. Cannon, J. et al. 2010, J. Proteome Res. 9, 3886-3890. These are termed "PRSMs" to distinguish them from matches to individual peptides. Here again the algorithms typically rely on a knowledge of the overall mass of the precursor and they are all premised on the notion that a comparison is made between observed protein spectra and predicted (in silico derived) protein spectra.

Several optimization approaches have been described in the art, for instance, the application of a precursor mass filtering (Geer, L.Y. et al. 2004, J. J Proteome Res. 3(5), 958-964) and the matching of the observed peptide fragments thereafter on a theoretical spectrum of merged B- and Y-ions. Other examples include, MASCOT, SEQUEST, X!TANDEM, SPECTRUM MILL, Andromeda, MS Amananda, CRUX. Additionally, a clustering of the observed fragment and the next matching can be performed (Frank, A.M. et al. 2008, J. Proteome Res. 7(1), 113-122) to improve the running time of the analysis in comparison with a database search. Alternatively, a search can be performed for spectral similarities between observed and identified spectra and spectra that are not identified (Wilhelm, T. etal. 2014, J. J Proteome Res. 13(9), 4002-4011), the core of which relies on a mathematical convolution of the spectral data. Each of these approaches, however, fail to adress one or more problems of the art.Similar challenges exist in the determination of the structure of other biomacromolecules such as nucleic acids and polysaccharides.

There is a need in the art for improved proteomics, genomics, transcriptomics, and glycomics methods.

### SUMMARY OF THE INVENTION

The methods provided herein address one or more drawbacks of the methods of the prior art.

The inventors have found a way to leverage accurate mass instrumentation with a theoretical fragment ion spectrum to be matched against an observed mass spectrum. Applied to proteins, a PRSM-style predicted spectrum can be matched against a peptide-scale spectrum. The biomacromolecules need not be in any way limited in terms of the digestion that produced them. In the context of proteins, an important advantage of the present methods is that the peptides need not be tryptic, they can even be endogenous, and there is no limitation on the PTMs which they may be manifesting, the fragmentation mechanism used or even the purity of the spectra. The methods provided herein are based on the ability to quickly recognize a series of peaks as having been produced by an arbitrary subsequence of the full proteome. This can be done with high specificity and sensitivity thanks to the accuracy of modern mass spectrometers. In particular embodiments, the speed comes from the application of infinite accuracy spectral convolution followed by a single-pass clustering technique. The result is an assignment of peaks from original, raw scan to a region of a specific biomacromolecule (so called hot-spot). The assignment may be reported with an associated p-value as the distribution of convolution scores follow a Poisson distribution. In some embodiments, the only pieces of input required in methods provided herein are: (a) raw spectra to be analyzed, i.e. observed mass spectra which stem from one or more mass spectroscopy experiments, (b) the biological sequence which is looked for, e.g. a FASTA file with the proteome of the target organism, and (c) a user-defined threshold (or clustering parameter) which corresponds to the accuracy of the mass spectrometer which was used to obtain the observed mass spectra of step (a).

More particularly, the invention provides computer-implemented methods for determining the presence of a protein as a biomacromolecule of an organism in a sample. The methods of the invention comprise the step of comparing an observed mass spectrum of the sample with a theoretical fragment ion spectrum comprising theoretical fragment ion masses of the biomacromolecule of interest.

The methods of the present invention can be applied to different types of mass spectroscopy techniques and the embodiments described herein serve as a proof of concept. Additionally, the methods can also be combined with other separation techniques (e.g. chromatography, mobility) and data tools (e.g. analysis, interpretation, representation, characterization) known in the art.

The methods comprise the step of obtaining a mass spectrum of the sample thereby obtaining a set of query peaks; subtracting the m/z value (i.e. the mass-charge ratio) of every query peak from the theoretical fragment ion masses of the macromolecules (for the organism of interest); clustering and scoring the resulting differences, thereby obtaining scores representative of the likelyhood of the presence of a specific biomacromolecule; and assigning the spectrum to a specific biomacromolecule, based on said scores, thereby identifying the presence of said biomacromolecules in said sample.

Said method comprises the steps of:
- obtaining an observed mass spectrum of the sample thereby obtaining a set of query peaks;
- subtracting the m/z value of every query peak from the theoretical fragment ion masses of the target proteome of said organism obtained by determining theoretical fragment ion spectra comprising theoretical fragment ion masses for the protein sequences of said target proteome;
- clustering and scoring the resulting differences, thereby obtaining scores representative of the likelyhood of the presence of a specific protein in said sample; and
- assigning the observed spectrum to a protein of said proteome, based on said scores, thereby identifying the presence of said protein in said sample.

In particular embodiments, the observed mass spectrum is obtained by tandem mass spectroscopy.

In particular embodiments, the methods comprise the step of obtaining a tandem mass spectrum of the sample thereby obtaining a set of query peaks; subtracting the m/z value (i.e. the mass-charge ratio) of every query peak from the theoretical fragment ion masses of the macromolecules (for the organism of interest); clustering and scoring the resulting differences, thereby obtaining scores representative of the likelyhood of the presence of a specific biomacromolecule; and assigning the spectrum to a specific biomacromolecule, based on said scores, thereby identifying the presence of said biomacromolecules in said sample.

In particular embodiments, where the biomacromolecule is a protein, said method comprises the steps of:
- obtaining a tandem mass spectrum of the sample thereby obtaining a set of query peaks;
- subtracting the m/z value of every query peak from the theoretical fragment ion masses of the target proteome of said organism obtained by determining theoretical fragment ion spectra comprising theoretical fragment ion masses for the protein sequences of said target proteome;
- clustering and scoring the resulting differences, thereby obtaining scores representative of the likelyhood of the presence of a specific protein in said sample; and
- respectively assigning the spectrum to a protein of said proteome, based on said scores, thereby identifying the presence of said protein in said sample.

In some embodiments, said theoretical fragment ion spectra are obtained by assuming that at least 25% of all possible ions, more preferably at least 75% of all possible ions, most preferably by assuming all possible ions are generated from the sequences in said proteome.

The method comprises generating theoretical ion masses for the target proteome and computing error intervals for every fragment ion mass based on the error tolerance of the mass spectrometry instrumentation.

In some embodiments, the method comprises selecting a theoretical fragment ion spectrum *i* corresponding to a given protein and comparing it to an observed fragment ion spectrum *j.*

In some embodiments, the method comprises, for every observed fragment mass, selecting a mass value p*y* from observed fragment spectrum *j,* selecting a mass value mx from theoretical fragment spectrum *i*, aligning the observed spectrum to the theoretical spectrum by
- computing a mass shift τxy = mx-py; and
- adjusting the observed fragment ion masses by adding τxy, such that the new mass of peak py now equals mx.

In some embodiments, the method further comprises searching for a pattern and scoring said pattern by a method comprising the steps of:
a) computing the number of fragment ion masses that coincide with the adjusted masses of the observed fragment ion spectrum given the precomputed error tolerance intervals (count*_{xy}*);
b) computing the sum of the intensities of the observed fragment ion masses coinciding with theoretical fragment ion masses (sum*_{xy}*).

In some embodiments, the method further comprises modelling the distribution of the number of coinciding fragment ions by a Poisson model and generating for each location a p-value for the probability of a match between an observed fragment ion spectrum and a (part of) a theoretical ion spectrum, and optionally correlating the local score distribution for additional confidence, wherein positions with a p-value smaller than a pre-determined significance level are regarded as statistically significant.

In some embodiments, the method further comprises annotating the observed fragment spectrum to show which peaks were matched by the theoretical ion fragments and updating the sequence to show which subsequence had observed matching ion fragments. In some embodiments, the theoretical fragment ion masses are adjusted by assuming a charge state z: the "theoretical fragment ion masses" are updated to "(theoretical fragment masses)/*z*".

In some embodiments, the sample comprises more than one biomacromolecule from said organism.

Further provided is a data-processing system comprising means configured for carrying out a method provided herein.

Further provided is a computer program product having instructions which when executed by a computing device or system cause the computing device or system to perform a method provided herein.

### BRIEF DESCRIPTION OF THE DRAWINGS

The following description of the figures of specific embodiments of the invention is merely exemplary in nature and is not intended to limit the present teachings, their application or uses.
**Fig. 1****:** Schematic illustration of how the methods and systems according to particular embodiments of the present invention investigate peptide spectra data against a protein sequence reference database according to a particular embodiment of the invention.
**Fig. 2****:** Schematic illustration of protein character strings converted into numeric sequences by converting every letter, to the mass of the predicted ion for the associated Amino Acid letter code. The resulting mass is appended onto a running total of ion masses, effectively resulting in a synthetic spectrum against which comparisons of real spectra can be made.
**Fig. 3****:** Exemplary scores for the extent to which an observed mass spectrum corresponds to a particular (part of) a theoretical mass spectrum. In particular, the figure shows how a given empirical (i.e. observed) spectrum against every possible theoretical start-stop position (i.e. peptide fragment) in every possible protein of the reference proteome.
**Fig. 4** The graphical representation of results obtained by the methods and systems according to particular embodiments of the invention includes: (a) annotation of observed ions with their matched pseudo-ions and (b) scoring of the protein sequences with the counts and/or intensities of their matching observed peaks.
**Fig. 5****.** An exemplary observed CID-spectrum of peptidic origin according to an embodiment of the invention
**Fig. 6** Matching pseudo-ion counts for both the b-ion series (a) and the y-ion series (b) according to an embodiment of the invention.
**Fig. 7** Poisson Model used in p-value estimation along with observed match to empirical count distribution according to an embodiment of the invention.

Throughout the figures, the following numbering is adhered to: 1 - spectrum collection; 2 - spectral matching; 3 - spectral assignment; 4 - protein database; 5 - theoretical fragment ion spectra; 6 - sequence coverage protein i.

### DETAILED DESCRIPTION

The present invention will be described with respect to particular embodiments but the invention is not limited thereto but only by the claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional elements or method steps. The terms "comprising", "comprises" and "comprised of" when referring to elements or method steps cited herein also include embodiments which "consist of" said elements or method steps cited herein. Furthermore, the terms first, second, third and the like as used herein are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

In the present disclosure (not part of the invention), the algorithms provided herein are mostly described in the context of protein sequencing. In particular, proteins and peptides, are bio-polymers that are composed of a well-defined alphabet. We have a 20-letter alphabet in the case of proteins and peptides. Any fragmentation technique for mass spectroscopy (MS), e.g. tandem MS, that produces reproducible fragment ions for the biopolymers will generate data that is compatible with the preent methods, which allows relating en observed fragment pattern to a text pattern. The present disclosure focuses on peptide to protein mapping.

The present inventors have identified a new way which allows for the identification of proteins as biomacromolecules in a sample based on the matching of peptide spectra. Generally, the method comprises the step of comparing an observed mass spectrum of a sample with a theoretical fragment ion spectrum.

In some embodiments, the observed spectrum is generated from a sample of a target organism. Also, accurate-mass clustering may be applied to said spectrum, for example by taking into account precomputed error intervals, as is detailed below. The observed spectra may correspond to a single peptide, or they may be chimeric or mixed (i.e. comprising ions from more than one peptide).

The theoretical fragment ion spectrum typically comprises theoretical fragment ion masses and theoretical fragment ion series.

In some embodiments, the observed mass spectrum is compared to the theoretical ion-series of every intact protein in the set of the particular proteins present in a target organism.

The method preferably involves comparing an observed mass spectrum with a theoretical fragement ion spectrum which comprises predicted ion series of every intact protein in the target organism's proteome. Preferably, the theoretical fragment ion spectrum is clustered before the comparison, wherein the clustering preferably takes into account the accuracy of the mass spectrometer by which the observed mass spectrum is obtained. This clustering step is explained in detail below. The result is an assignment of peaks from the spectra to sub-sequences in the protein database.

The methods according to the invention typically require only an observed mass spectrum and a FASTA file of the reference proteome. In particular, any information about precursor masses, digestion protocols, fragmentation techniques, expected PTMs (post-translational modifications) or mutations is typically not required.

The methods allow for the interpretation of mass spectrometry-generated fragment ion spectra of amino acid sequences based on a search against a protein database that does not depend on user defined parameters. In the context of protein sequencing, the only two parameters are inferred from the experimental set-up and include the protein database (defined by the organism under scrutiny) and the mass accuracy (defined by the mass spectrometer).

The invention thus provides a computer-implemented method for determining the presence of a biomacromolecule of an organism in a sample wherein the method comprises the step of comparing an observed mass spectrum of the sample with a theoretical fragment ion spectrum comprising theoretical fragment ion masses. In some embodiments, the comparison involves spectral convolution of the observed mass spectrum with the theoretical fragment ion spectrum, wherein said biomacromolecule is a proteinThe method may comprise comparing the obtained mass spectrum of the sample with the predicted a/x, b/y, and/or c/z series of the target proteome.

More particularly, the biomacromolecule is a protein and the method comprises the steps of:
- obtaining a tandem mass spectrum of the sample thereby obtaining a set of query peaks;
- subtracting the m/z value of every query peak from the theoretical fragment ion masses of the target proteome of said organism obtained by determining theoretical fragment ion spectra comprising theoretical fragment ion masses for the protein sequences of said target proteome;
- clustering and scoring the resulting differences, thereby obtaining scores representative of the likelyhood of the presence of a specific protein in said sample; and
- respectively assigning the spectrum to a protein of said proteome based on said scores, thereby identifying the presence of said protein in said sample.

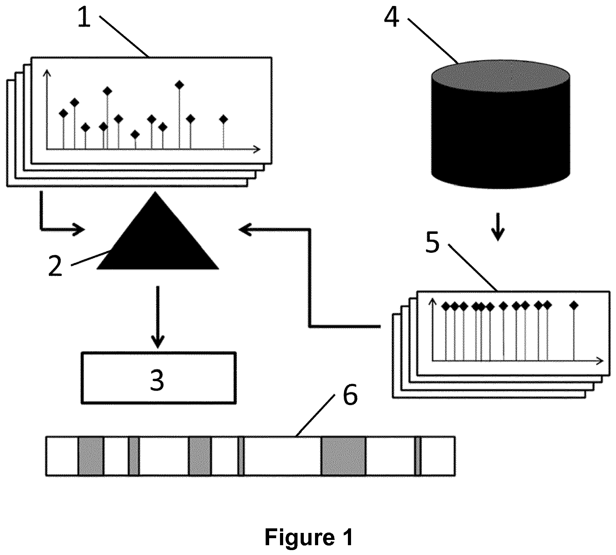

The principle is illustrated in Figure 1 for the case in which the biomacromolecule is a protein. As described herein, tandem mass spectrometry refers to a particular approach wherein distinct ions of interest are selected based on their m/z value from the first round of mass spectrometry and are fragmented by a number of methods of dissociation (e.g. collisions with (high energy) inert gas, electron-transfer, electron-capture, etc.). These fragments are then separated based on their individual m/z ratios in a second round of mass spectrometry.

In a particular, where the biomacromolecule is a protein, said method comprises the steps of:
- obtaining an observed mass spectrum of the sample thereby obtaining a set of query peaks;
- subtracting the m/z value of every query peak from the theoretical fragment ion masses of the target proteome of said organism obtained by determining theoretical fragment ion spectra comprising theoretical fragment ion masses for the protein sequences of said target proteome;
- clustering and scoring the resulting differences, thereby obtaining scores representative of the likelyhood of the presence of a specific protein in said sample; and
- respectively assigning the observed mass spectrum to a protein of said proteome, based on said scores, thereby identifying the presence of said protein in said sample.

As described herein, mass spectrometry refers to a spectroscopic technique of measuring the mass-to-charge ratio of ions to identify and quantify molecules in simple and complex mixtures.

In particular embodiments, the characterization procedure comprises the steps of collecting the spectrum (1), and spectral matching (2) so as to obtain an spectral assignment (3). In the spectrum collection step, an observed mass spectrum of a peptide is obtained. For the spectral matching, theoretical fragment ion spectra (5) are obtained from a protein database (4). The observed mass spectrum is compared to theoretical fragment ion spectra (5) in a spectral matching step (2). After the spectral matching step (2), the observed mass spectrum is assigned to a protein sequence. By repeating the above procedure for various observed mass spectra, proteins are sequenced.

Preferably, the theoretical fragment ion spectra comprise all fragment ions which might be created from a proteome in a specific mass spectrometry fragmentation. However, the theoretical fragment ion spectra might comprise more theoretical fragment ions as well. For example, when the method is applied to the study of proteins, the theoretical fragment ion spectra might comprise all a-,b-,c-,x-,y-, and z-series of the target proteome even though it is known that the fragmentation technique used to obtain the observed fragment ion spectrum only produces b- and y- ions. Also, the theoretical fragment ion spectra may not comprise all fragment ions which occur in the sample. For example, the sample may comprise protein fragments comprising post-translational modifications and/or mutations which do not occur in the theoretical fragment ion spectra.

Accordingly, in particular embodiments said theoretical fragment ion spectra are respectively obtained by assuming at least 25% of all possible ions, more preferably at least 75% of all possible ions, most preferably by assuming about all possible ions generated from the protein sequences in said proteome. For amino acids, the recitation "all possible ions" as used herein refers to a set of ions which may comprise all possible ion fragments starting from the o-terminus and all possible ion fragments starting from the n-terminus. For proteins, the "all possible ions" may comprise all a- and x- fragments, all b- and y- fragments, and/or all c- and z- fragments. For proteins, the mass differences between different ions comprised in the "all possible ions" are preferably equal to the mass of an integer number of amino acid residues.

The methods provided herein thus involve the analysis of a mass spectrum obtained from a sample. Methods of mass spectrometry (MS) and tools for performing them are known in the art and include collision-induced dissociation (CID), also known as "collisionally activated dissociation" (CAD). The method induces fragments of molecular ions in the gas phase. The molecular ions are accelerated and allowed to collide with neutral molecules (such as helium, nitrogen or argon). In the collision some of the kinetic energy is converted into internal energy which results in bond breakage and the fragmentation of the molecular ion into smaller fragments. These fragment ions can then be analyzed by a tandem mass spectrometry. Examples of mass spectrometers include Triple quadrupole mass spectrometers, Fourier transform ion cyclotron resonance, Sustained off-resonance irradiation collision-induced dissociation (SORI-CID) spectrometers and higher-energy collisional dissociation (HCD) or "orbitrap" mass spectrometers. The methods provided herein may include the step of submitting the sample to mass spectrometry but typically start out from the resulting spectrum which is obtained. This is referred to herein as the "query spectrum S" or "S". In particular embodiments the method is a computer-implemented method.

The sample on which the mass spectrometry analysis is performed is not critical to the methods provided herein. The sample submitted for MS analysis may be solid, liquid, or gas. In light of the analysis method however, the biomacromolecule in the sample can typically be attributed to an organism (also referred to as the target organism herein). When the biomacromolecule is a protein, this allows for the comparison with the proteome of said organism in the methods provided herein.

The methods provided herein may involve a pre-processing step wherein theoretical fragment ion spectra are obtained by assuming at least 25% of all possible ions, more preferably at least 75% of all possible ions, most preferably by assuming all posible ions in the proteome.

The methods provided herein may involve a pre-processing step wherein the complete a-, b, or c- and respectively x- y- or z- series for every protein in the proteome of the organism of interest is calculated. This step will be required only once for a given organism and can in most cases be ensured based on information retrieved from Genbank (http://www.ncbi.nlm.nih.gov/genbank/) or other publicly accessible sequence databases. The data obtained can then be distributed across the nodes of a multiprocessor or across the cores of a multi-core chipset for a trivial speedup (so called embarrassingly simple parallelization).

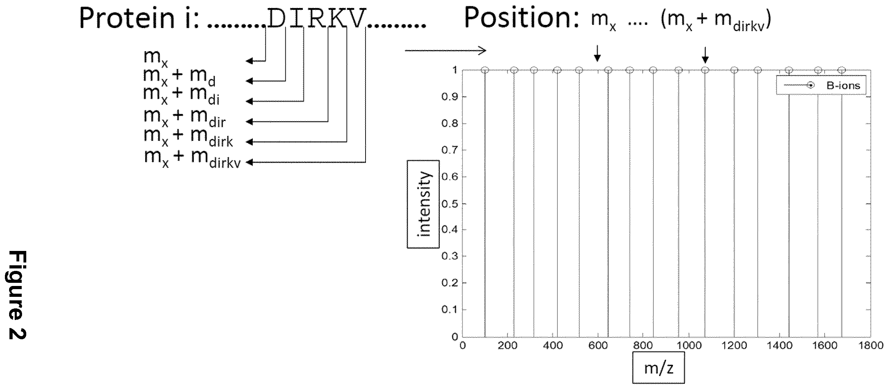

Indeed, for proteins and in order to allow comparison with a query spectrum, the proteome may be converted into a synthetic spectrum by assuming all possible a-, b, or c- and respectively x- y- or z- ions that can be generated from these proteins. Each of the protein sequences in the proteome are converted into a list of pseudo-masses. In this regard, a particular pseudo mass need not correspond to a specific observed ion-value, but its value relative to the other pseudo-masses implies its unique position in the protein sequence. In doing so, a theoretic spectrum is produced in silico. This is illustrated in figure 2.

The theoretical fragment ion spectra may be obtained by assuming all possible a-, b, or c- and/or x- y- or z- generated from the protein sequences in said proteome. The use of all possible a-, b, or c- and/or x- y- or z- independent of certain assumptions of digestion of the protein in the sample allows for a more accurate analysis which can take into account possible (unknown) modifications of the protein.

Accordingly, in the theoretical fragment ion spectra each of the proteins of the proteome is associated with M theoretical a-, b, or c- and/or x- y- or z-. Potentially, the observed fragment ion masses can be produced by any sub-pattern in the protein sequence. Therefore, M possible starting locations in the protein sequence may be considered to search for a co-occurring pattern that explains the fragment ions in the observed peptide spectrum. When the origin of the observed fragment ion masses are unknown, every fragment ion in the spectrum may be considered as a potential product of the protein at a particular starting location.

Methods according to the present disclosure should be contrasted with prior art methods such as the methods by Wilhelm et al (as referenced in the Background section). Wilhelm et al. tries to explain modifications in observed fragment spectra. Doing so they first apply a biclustering approach to group spectra that share a subset of fragments. Second, they will look at particular mass differences between two bicluster groups to screen for a potential shift of the fragment peaks. So Wilhelm et al looks for structure in the data, but does not identify the spectra. Conversely, the present methods involve comparing an observed mass spectrum of a sample with a theoretical fragment ion spectrum. Thus the present methods may entail searching for patterns, or equivalently subsets, or equivalently mass differences, or equivalently item sets, etc., between an observed mass spectrum of a protein fragment in a sample and a theoretical fragment ion spectrum, or in other words a virtual protein fragment spectrum. Both methods have in common that they may be said to look at patterns that are shared between peak lists, i.e., spectral alignment, but the implementation and application is different. For example, to map peptides on proteins, the present methods can be said to involve disconnecting the b- and y-ion series. Such thing is not possible in the application of Wilhelm et al. as they stick with observed fragment data.

The methods comprise generating theoretical ion masses for the target proteome at the a-, b, or c-ion bond and the x- y- or z-ion bond respectively, and computing error intervals for every fragment ion mass based on the error tolerance of the mass spectrometry instrumentation. Indeed, in order to ensure that the theoretical masses can be adequately compared to the peaks in the spectra obtained, the potential error margin of the mass spectrometer used can be taken into consideration.

In a next step, the peaks of the query spectrum S may be compared with the theoretical a-, b, or c- and/or respectively x- y- or z-series values. This is preferably done by spectral convolution. Preferably, the user can specify the mass precision to be considered for this mathematical operation. For every query spectrum S and every protein a/x, b/y, or c/z-series, the m/z value of every query peak from every predicted a/x, b/y, or c/z value is substracted. This step can be done spearately, or by matching pooled a/x, b/y, or c/z series. This step is also trivially parallelizable. The data can remain in its original floating point representation or it can be rounded. However, care must be take to round well beyond the accuracy of the instrument. In particular embodiments, rounding is pursued only in cases where the resulting speed gains justify the move away from full floating point representation.

The resulting differences may then be sorted by DBSCAN-like Clustering, as described by Martin Ester, Hans-peter Kriegel, Jörg Sander, and Xiaowei Xu, A density-based algorithm for discorvering clusters in large spatial databases with noise, 7th International converence on Data Warehousing and Knowledge Discovery, 1996, pp. 226-231. The sorted deltas are then traversed with every delta assumed to be the initiator of a cluster. If the succeeding delta is within 2*ε* of the previous delta (where *ε* is the error tolerance of the instrument in ppm) the succeeding delta is added to the cluster and the delta value of the cluster is updated by a central value of the delta's composing the cluster (mean, median, mode, max, etc.). It is noted that this step is also parallelizable though not trivially. Accordingly, in particular embodiments, the methods comprise generating theoretical ion masses for a target proteome, and computing error intervals for every fragment ion mass based on the error tolerance of the mass spectrometry instrumentation. This allows grouping theoretical ion masses which are separated by less than the error tolerance.

In a next step, the methods may encompass a significance analysis and biomacromolecule assignment. The convolution step described above produces a clusters that contain a varying number of delta values that correspond to putative peaks "shifted" relative to the predicted total protein spectrum. The significance of the correspondence can be determined using a formal p-value based on an assumed (modified) Poisson distribution that is suited to deal with the count statistics produced by the DBSCAN algorithm. The modification of the distribution is trivial, in that a normal Poisson distribution entails "zero counts" as a possibility, while in the present methods there are no zero counts as a peak is always matched between the virtual protein and observed peptide peptides. For this reason, the "one counts" are inflated.

The peaks participating in delta clusters considered significant are then assigned to the relevant biomacromolecule entry.

Where the spectrum is from a mixed sample (unintentionally, e.g., chimeric spectra or by design, e.g., in the data-independent acquisition (DIA) experimental paradigm), different subsets of the peaks in the spectrum will be assigned to different biomacromolecules. An example of a practical implementation of how these steps of spectral convolution, clustering and assignment may be carried out is detailed below for proteins.

First, a theoretical fragment ion spectrum i is selected corresponding to a given protein or chromosome and it is compared to an observed fragment ion spectrum j.

In particular embodiments, the theoretical fragment ion masses are adjusted by assuming a charge state *z*, i.e. the "theoretical fragment ion masses" are updated to "(theoretical fragment masses)/*z*". However, this latter step is optional.

In particular embodiments, the method then comprises selecting a mass value py from observed fragment spectrum j, selecting a mass value mx from theoretical fragment spectrum *i*, aligning the observed spectrum to the theoretical spectrum by computing a mass shift τxy = mx-py and adjusting the observed fragment ion masses by adding τxy, such that the new mass of peak py now equals mx. This can be interpreted as searching for patterns between observed and theoretical data that can be aligned for the annotation of observed fragment ions. In this context, we note that Wilhelm et al (referred to in the background section) looks for mass differences to find patterns that explain modifications. Both the present methods and the disclosure of Wilhelm et al use the terminology of a mass shift, but the interpretation is different. The term "mass shift" is commodity in mass spectrometry.

In a next step the methods may comprise searching for a pattern and scoring it. In particular embodiments this involves:
- Computing the number of fragment ion masses that coincide with the adjusted masses of the observed fragment ion spectrum given precomputed error tolerance intervals (count*_{xy}*); and
- Computing the sum of the intensities of the observed fragment ion masses coinciding with theoretical fragment ion masses (sum*_{xy}*).

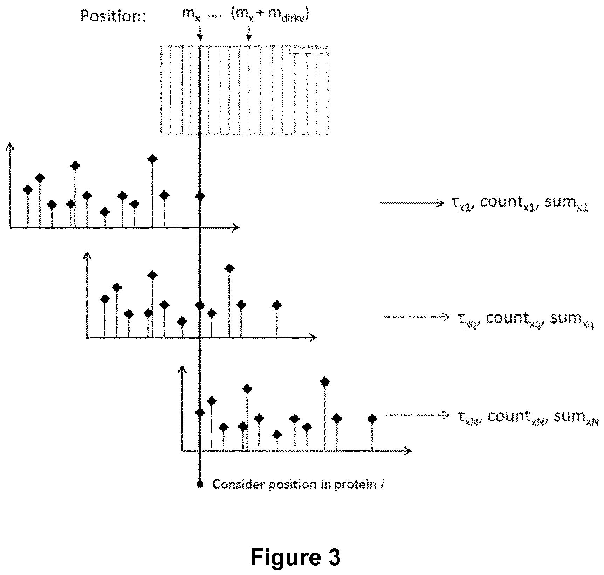

This is repeated for every possible theoretical position in every possible biomacromolecule of the set of reference biomacromolecules. For proteins, the set of reference biomacromolecules corresponds to the reference proteome (Figure 3).

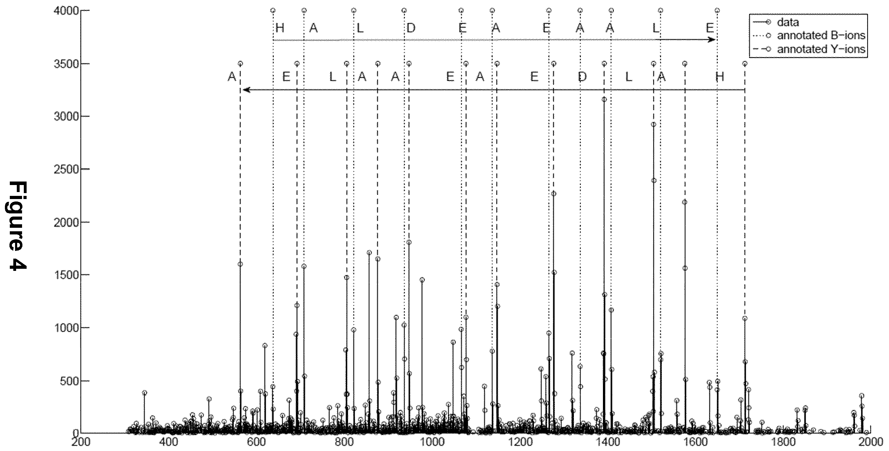

The distribution of the number of coinciding fragment ions is then preferably modelled by a Poisson model whereby for each location a p-value is generated, the p-value representing the probability of a match between an observed fragment ion spectrum and a (part of) a theoretical ion spectrum. Optionally, the local score is correlated for additional confidence. In particular embodiments, positions with a p-value smaller than a pre-determined significance level (for example a significance level of 0.05) are regarded as statistically significant. Thereafter the observed fragment spectrum can be annotated to show which peaks are matched by the theoretical ion fragments (shown for proteins in Figure 4a). Similarly, the sequence can be updated to show which subsequence has observed ion fragments (shown for proteins in Figure 4b).

The above process can be repeated for every combination of observed ion spectra and theoretical fragment spectra. This makes it possible to identify the presence of a given biomacromolecule, for example a given protein, in the sample. In particular embodiments, the sample comprises more than one biomacromolecule from an organism, wherein the one or more biomacromolecules are proteins.

The application of the methods provided herein are numerous. Indeed, the specific advantages of the methods provided herein allow for analysis of sets of biomacromolecules, for example for analysis of the proteome, in conditions where prior art methods have failed, due to limitations on the nature of the sample and/or the need for prior knowledge of the starting material. The following are exemplary applications of the provided methods.

**Post Translational Modifications/Mutations (PTM):** while different approaches to the problem of proteins comprising PTM have been published, the present methods broaden the applicability of PTM/Mutation tolerant search by eliminating the requirement for an enzymatically-constrained peptide population. In addition, by avoiding the reliance on a complementary reference search, the present methods preserve the ability to provide straightforward p-values since they do not involve complex, difficult to analyze iterative searches such as employed by many "tolerant" search techniques (which are usually required to eschew probabilistic characterization of their results). Accordingly, in particular embodiments, the methods are performed to identify proteins which may be subject to PTM.

**Endogenous Peptidomics:** Since the methods provided herein do not require prior knowledge of the process which generated the peptide population being analyzed, they are ideal for endogenous peptidomics. Endogenous peptides are often modified in-vivo prior to full biological activation. Since the methods provided herein do not require prior knowledge of these modifications they have the unique advantage of their ability to detect this challenging peptide population. Accordingly, in particular embodiments, the methods are performed to identify endogenous peptides.

**Searching Chimeric Spectra:** Chimeric spectra represent another challenging case for typical search engines (which usually address them through an iterative scheme which, as previously mentioned, causes difficulty in the calculation of valid p-values). The present methods, by not requiring pure spectra, provide a natural match for such spectra. Accordingly, in particular embodiments, the methods are performed to identify biomacromolecules present in a sample generating an chimeric spectrum.

**Data Independent Acquisition:** A particularly competitive match for the methods and systems according to the present invention is the analysis of DIA-based spectra. These are well-known for their complexity since multiple precursors are fragmented concurrently. The present methods will do particularly well in this situation since it does not require knowledge of the precursors (i.e. there is no need for the low-energy scan - in the case of Waters^{®} data - or the information about the SWATH^{™} window in the case of ABSciex^{®} data).

**Label Free (fragment-based) Quantification:** DIA-based quantitative proteomics has been an appealing prospect thanks to the freedom from the stochasticity of precursor selection. However, the difficulty in the identification of the resulting spectra has limited its spread. By potentially eliminating the need for precursor information while still maintaining excellent identifiability, the present methods enable fragment-based quantification on much cheaper instrumentation then is currently feasible. This allows quantitative proteomics to be performed on devices capable of accurate mass but limited to all-ion fragmentation (hence much cheaper than the typical instruments used for quantitative proteomics).

**Spectral library matching:** In spectral library matching an unknown observed peptide spectrum is queried against a library of high quality fragment spectra for which the petide sequence assignment is known. In the approach according to the present invention, spectral library searching is tolerant agianst unspecified PTMs.

**Invariant with respect to the fragmentation principle:** Methods according to the present inventionare invariant of the type of fragmentation and works equally well for collision-induced dissociation (CID), electron-capture dissociation (ECD), electron-transfer dissociation (ETD), negative electron-transfer dissociation (NETD), electron-detachment dissociation (EDD), photodissociation, particularly infrared multiphoton dissociation (IRMPD) and blackbody infrared radiative dissociation (BIRD), surface-induced dissociation (SID), Higher-energy C-trap dissociation (HCD), charge remote fragmentation. For these applications it is not required to define new fragment ion series, since the mass difference between consecutive fragment ions remains fixed for all ion fragment types.

In particular embodiments, the methods provided herein are computer-implemented methods.

Accordingly, in some embodiments, the application provides computer-implemented methods for determining the presence of a biomacromolecule of an organism in a sample, wherein the biomacromolecule is a protein, the method comprising the steps of:
a. receiving a biomacromolecule database comprising the biomacromolecule sequences, for example of the proteome, of said organism;
b. determining theoretical fragment ion spectra for the protein sequences, the theoretical fragment ion spectra comprising M theoretical fragment ion masses, the theoretical fragment ion spectra corresponding to biomacromolecules *i*;
c. receiving an observed sample spectrum corresponding, the observed spectrum comprising N observed fragment ion masses;
d. matching the observed sample spectrum to the theoretical fragment ion spectrum or a part thereof by clustered spectral convolution; and
e. identifying a protein present in said sample based thereon.

In particular embodiments, the methods are designed to take into consideration the error tolerance of the mass spectrometry method. Accordingly, in particular embodiments, the computer-implemented methods further comprise the step of receiving an error tolerance, which can then be taken into consideration in the methods as described herein.

Further provided herein is a computer readable medium having stored thereon instructions which when executed by a computing device or system cause the computing device or system to perform the steps of a method provided herein.

The application further provides data-processing systems comprising means configured for carrying out the methods provided herein.

The application further provides computer program products having instructions which when executed by a computing device or system cause the computing device or system to perform a method as described herein.

The application further provides data streams which are representative of a computer program having instructions which when executed by a computing device or system cause the computing device or system to perform the methods as described herein.

### Examples

### 1. Identification of a protein in a CID spectrum

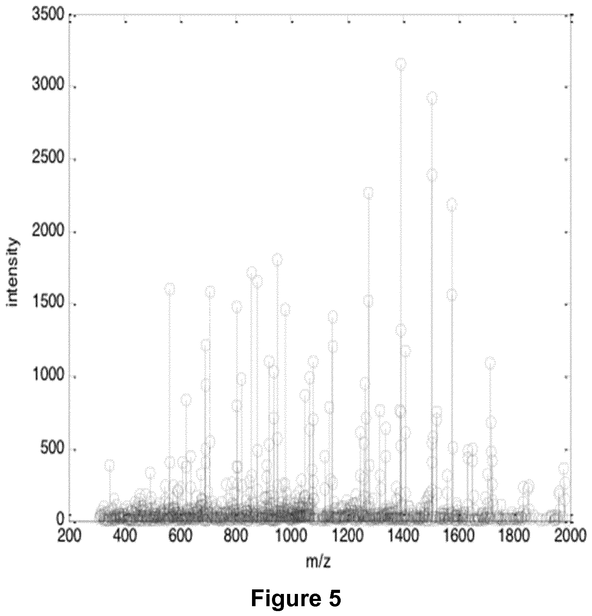

The CID spectrum in Figure 5 is searched against a protein with the sequence:

IIDEWRKKTDDLAAELDGAQRDLRNTSTDLFKAKNAQEELAEVVEGL
RRENKSLSQEIKDLTDQLGEGGRSVHEMQKIIRRLEIEKEELQHALD
EAEAALEAEESKVLRAQVEVSQIRSEIEKRIQEKEEEFENTRKNHAR
ALESMQASLE (SEQ ID NO: 1)

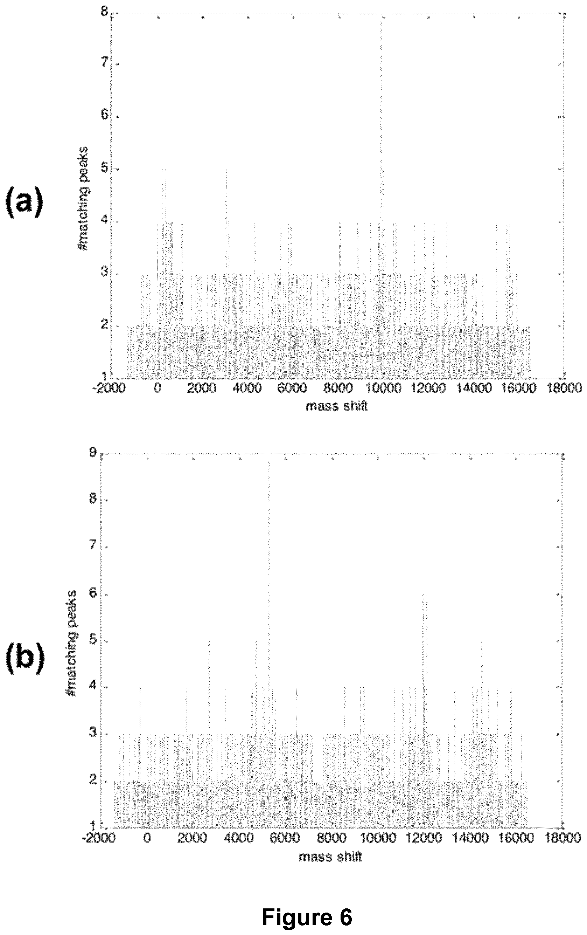

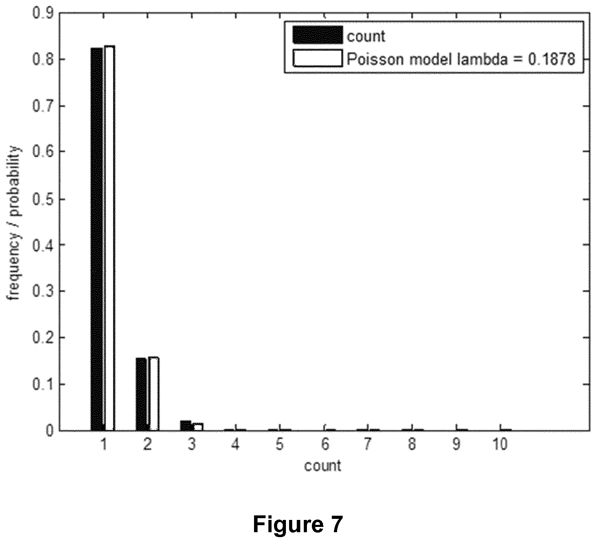

Applying the methods according to the present invention with an error tolerance of 0.05 Da yields b-ion (1+) and y-ion (1+) matching pseudo-ion counts as seen in Figure 6a and 6b. The count score is modelled by a Poisson distribution (see Figure 7) and p-values are generated for each count. Counts of 8 and 9 as are seen in the y-ion and b-ion series respectively are statistically significant and lead to the following annotation:
Result in terms of sequence:
1. B-ion result: LQHALDEAEAALEAEE [p-val: 6.8e-13] (SEQ ID NO: 2)
2. Y-ion result: LQHALDEAEAALEAEE [p-val: 3.3e-11] (SEQ ID NO: 3)
are confirmed by the SEQUEST algorithm as: EELQHALDEAEAALEAEESK (SEQ ID NO:4)

### 2. Exemplary workflow

The methods provided herein are applied to protein characterization, as schematically illustrated in Fig. 1. In particular, the characterization procedure comprises the step of spectrum collection (1). In the spectrum collection step, an observed mass spectrum of a peptide is obtained. The observed mass spectrum is compared to theoretical fragment ion spectra (5) in a spectral matching step (2). Prior to the spectral matching step (2), the theoretical fragment ion spectra (5) were derived from a protein database (4). After the spectral matching step (2), the observed mass spectrum is assigned to a protein sequence. By repeating the above procedure for various observed mass spectra, proteins are sequenced. The sequence coverage for a specific protein I (6) is schematically shown at the lower end of the figure

## Claims

1. A computer-implemented method for determining the presence of a biomacromolecule of an organism in a sample comprising the step of comparing an observed mass spectrum of the sample with a theoretical fragment ion spectrum comprising theoretical fragment ion masses; wherein said biomacromolecule is a protein, and wherein said method comprises the steps of:
- obtaining an observed mass spectrum of the sample thereby obtaining a set of query peaks;
- subtracting the m/z value of every query peak from the theoretical fragment ion masses of a target proteome of said organism obtained by determining theoretical fragment ion spectra comprising theoretical fragment ion masses for the protein sequence of said target proteome;
- clustering and scoring the resulting differences, thereby obtaining scores representative of the likelyhood of the presence of a specific protein in said sample; and
- assigning the observed mass spectrum to a protein of said proteome based on said scores, thereby identifying the presence of said protein in said sample;
wherein said theoretical fragment ion spectra are obtained;
- by generating complete theoretical fragment ion masses for the target proteome sequence at a-, b-, or c-ion bond and - x-, y-, or z-ion bond respectively and;
- computing error intervals for every fragment ion mass based on error tolerance of the mass spectrometry instrumentation.

2. The method according to claim 1 wherein the observed mass spectrum is obtained by tandem mass spectroscopy.

3. The method according to claim any one of claims 1 to 2, wherein said theoretical fragment ion spectrum is obtained in a pre-processing step
- by calculating the complete a-, b-, c- and respectively x-, y-, z- series of every protein in the proteome of the organism of interest.

4. The method according to claims 1 to 3, which comprises selecting a theoretical fragment ion spectrum *i* corresponding to a given protein and comparing it to an observed fragment ion spectrum *j.*

5. The method according to claim 4 comprising, for every observed fragment mass, selecting a mass value py from observed fragment spectrum *j*, selecting a mass value mx from theoretical fragment spectrum *i*, aligning the observed spectrum to the theoretical spectrum by
- computing a mass shift τxy = mx-py; and
- adjusting the observed fragment ion masses by adding τxy, such that the new mass of peak py now equals mx.

6. The method according to claim 5 which further comprises searching for a pattern and scoring said pattern by a method comprising the steps of
a) Computing the number of fragment ion masses that coincide with the adjusted masses of the observed fragment ion spectrum given the precomputed error tolerance intervals (count*_{xy}*).
b) Computing the sum of the intensities of the observed fragment ion masses coinciding with theoretical fragment ion masses (sum*_{xy}*).

7. The method of claim 6 which further comprises modelling the distribution of the number of coinciding fragment ions by a Poisson model and generating for each location a p-value for the probability of a match between an observed fragment ion spectrum and a (part of) a theoretical ion spectrum, and optionally correlating the local score distribution for additional confidence, wherein positions with a p-value smaller than a pre-determined significance level are regarded as statistically significant.

8. The method of claim 6 or 7 which further comprises annotating the observed fragment spectrum to show which peaks were matched by the theoretical ion fragments and updating the sequence to show which subsequence had observed matching ion fragments.

9. The method according to any one of claims 1 to 8, wherein the theoretical fragment ion masses are adjusted by assuming a charge state z: the "theoretical fragment ion masses" are updated to "(theoretical fragment masses)/*z*".

10. The method according to any one of claims 1 to 9, wherein the sample comprises more than one biomacromolecule from said organism.

11. A data-processing system comprising means configured for carrying out a method according to any one of claims 1 to 10.

12. A computer program product having instructions which when executed by a computing device or system cause the computing device or system to perform a method according to any one of claims 1 to 10.

## Patentansprüche

1. Computerimplementiertes Verfahren zur Bestimmung des Vorhandenseins eines Biomakromoleküls eines Organismus in einer Probe, umfassend den Schritt des Vergleichens eines beobachteten Massenspektrums der Probe mit einem theoretischen Fragmentionenspektrum, das theoretische Fragmentionenmassen umfasst; wobei das Biomakromolekül ein Protein ist, und wobei das Verfahren die folgenden Schritte umfasst:
- Erhalten eines beobachteten Massenspektrums der Probe, wodurch eine Reihe von Abfragepeaks erhalten wird;
- Subtrahieren des m/z-Werts jedes Abfragepeaks von den theoretischen Fragmentionenmassen eines Zielproteoms des genannten Organismus, die durch Bestimmen theoretischer Fragmentionenspektren erhalten werden, die theoretische Fragmentionenmassen für die Proteinsequenz des genannten Zielproteoms umfassen;
- Gruppierung und Bewertung der sich ergebenden Unterschiede, um Ergebnisse zu erhalten, die repräsentativ für die Wahrscheinlichkeit des Vorhandenseins eines spezifischen Proteins in der Probe sind; und
- Zuordnen des beobachteten Massenspektrums zu einem Protein des Proteoms basierend auf den Bewertungen, wodurch das Vorhandensein des Proteins in der Probe identifiziert wird;
wobei die theoretischen Fragmentionenspektren erhalten werden;
- durch Erzeugung vollständiger theoretischer Fragmentionenmassen für die Zielproteomsequenz bei a-, b- oder c-Ionenbindung bzw. - x-, y- oder z-Ionenbindung; und
- Berechnung von Fehlerintervallen für jede Fragmentionenmasse basierend auf der Fehlertoleranz der Massenspektrometrieinstrumente.

2. Verfahren nach Anspruch 1, wobei das beobachtete Massenspektrum durch Tandemmassenspektroskopie erhalten wird.

3. Verfahren nach Anspruch auf einen der Ansprüche 1 bis 2, wobei das theoretische Fragmentionenspektrum in einem Vorverarbeitungsschritt erhalten wird
- durch Berechnung der vollständigen a-, b-, c- bzw. x-, y-, z- Reihe jedes Proteins im Proteom des betreffenden Organismus.

4. Verfahren nach den Ansprüchen 1 bis 3, das die Auswahl eines theoretischen Fragmentionenspektrums i, das einem gegebenen Protein entspricht, und den Vergleich mit einem beobachteten Fragmentionenspektrum j umfasst.

5. Verfahren nach Anspruch 4, umfassend für jede beobachtete Fragmentmasse die Auswahl eines Massenwerts py aus dem beobachteten Fragmentspektrum *j*, die Auswahl eines Massenwerts mx aus dem theoretischen Fragmentspektrum *i*, die Ausrichtung des beobachteten Spektrums auf das theoretische Spektrum durch
- Berechnen einer Massenverschiebung τxy = mx-py ; und
- Anpassung der beobachteten Fragmentionenmassen durch Zugabe von τxy, so dass die neue Masse des Peaks py jetzt mx entspricht.

6. Verfahren nach Anspruch 5, das ferner das Suchen nach einem Muster und die Bewertung des Musters durch ein Verfahren umfasst, das die folgenden Schritte umfasst:
a) Berechnung der Anzahl der Fragmentionenmassen, die mit den bereinigten Massen des beobachteten Fragmentionenspektrums unter Berücksichtigung der vorberechneten Fehlertoleranzintervalle (Anzahl_{xy} ) zusammenfallen.
b) Berechnung der Summe der Intensitäten der beobachteten Fragmentionenmassen, die mit den theoretischen Fragmentionenmassen zusammenfallen (Summe_{xy} ).

7. Verfahren nach Anspruch 6, das weiterhin das Modellieren der Verteilung der Anzahl zusammenfallender Fragmentionen durch ein Poisson-Modell und das Erzeugen eines p-Werts für jeden Ort für die Wahrscheinlichkeit einer Übereinstimmung zwischen einem beobachteten Fragmentionenspektrum und einem (Teil von) a eines theoretischen Ionenspektrums umfasst und optional Korrelation der lokalen Bewertungsverteilung für zusätzliche Sicherheit, wobei Positionen mit einem p-Wert kleiner als ein vorab festgelegtes Signifikanzniveau als statistisch signifikant angesehen werden.

8. Verfahren nach Anspruch 6 oder 7, das ferner die Kommentierung des beobachteten Fragmentspektrums umfasst, um zu zeigen, welche Peaks von den theoretischen Ionenfragmenten ausgeglichen wurden, und die Aktualisierung der Sequenz, um zu zeigen, welche Subsequenz übereinstimmende Ionenfragmente beobachtet hat.

9. Verfahren nach einem der Ansprüche 1 bis 8, wobei die theoretischen Fragmentionenmassen unter Annahme eines Ladungszustands z angepasst werden: die "theoretischen Fragmentionenmassen" werden auf "(theoretische Fragmentmassen)/z" aktualisiert.

10. Verfahren nach einem der Ansprüche 1 bis 9, wobei die Probe mehr als ein Biomakromolekül aus dem Organismus umfasst.

11. Datenverarbeitungssystem mit Mitteln, die zur Durchführung eines Verfahrens nach einem der Ansprüche 1 bis 10 eingerichtet sind.

12. Computerprogrammprodukt mit Anweisungen, die, wenn sie von einer Recheneinrichtung oder einem Computersystem ausgeführt werden, bewirken, dass die Recheneinrichtung oder das Computersystem ein Verfahren nach einem der Ansprüche 1 bis 10 durchführt.

## Revendications

1. Procédé implémenté sur ordinateur, visant à déterminer la présence d'une macromolécule biologique d'un organisme dans un échantillon, et comportant une étape de comparaison d'un spectre de masse observé de l'échantillon avec un spectre d'ions fragmentaires théorique comprenant des masses d'ions fragmentaires théoriques, dans lequel procédé la macromolécule biologique est une protéine, et lequel procédé comporte les étapes suivantes :
- obtenir un spectre de masse observé de l'échantillon, et par là, obtenir un ensemble de pics-questions,
- soustraire la valeur de m/z de chaque pic-question des masses d'ions fragmentaires théoriques d'un protéome cible dudit organisme, obtenues par détermination de spectres d'ions fragmentaires théoriques comprenant des masses d'ions fragmentaires théoriques pour la séquence protéique dudit protéome cible,
- rassembler et coter les différences résultantes, et par là, obtenir des cotes représentant la vraisemblance de la présence d'une protéine particulière dans ledit échantillon,
- et attribuer le spectre de masse observé à une protéine dudit protéome en se basant sur ces cotes, et par là, détecter la présence de cette protéine dans ledit échantillon,
étant entendu qu'on a obtenu lesdits spectres d'ions fragmentaires théoriques
- en générant un ensemble complet des masses d'ions fragmentaires théoriques pour la séquence de protéome cible, respectivement au niveau de la liaison d'ion a, b ou c et de la liaison d'ion x, y ou z,
- et en calculant des intervalles d'erreur pour chaque masse d'ion fragmentaire, fondés sur la tolérance d'erreur de l'instrument de spectrométrie de masse.

2. Procédé conforme à la revendication 1, dans lequel le spectre de masse observé est obtenu par spectroscopie de masse en tandem.

3. Procédé conforme à l'une des revendications 1 et 2, dans lequel on obtient ledit spectre d'ions fragmentaires théoriques, au cours d'une étape préalable, en calculant les séries a, b, c et respectivement x, y, z complètes de chaque protéine présente dans le protéome de l'organisme auquel on s'intéresse.

4. Procédé conforme à l'une des revendications 1 à 3, qui comporte le fait de choisir un spectre *i* d'ions fragmentaires théorique correspondant à une protéine donnée et le fait de le comparer à un spectre d'ions fragmentaires observé *j.*

5. Procédé conforme à la revendication 4, qui comporte, pour chaque masse de fragment observée, le fait de choisir une valeur py de masse dans le spectre de fragments observé *j*, le fait de choisir une valeur mx de masse dans le spectre de fragments théorique *i*, et le fait d'aligner le spectre observé sur le spectre théorique
- en calculant un décalage de masse txy = mx - py,
- et en ajustant les masses d'ions fragmentaires observées en y ajoutant txy, de telle sorte que la nouvelle masse du pic py est dorénavant égale à mx.

6. Procédé conforme à la revendication 5, qui comporte en outre le fait de chercher un schéma et de coter ce schéma selon un procédé comportant les étapes consistant :
a) à calculer le nombre de masses d'ions fragmentaires qui coïncident avec les masses ajustées du spectre d'ions fragmentaires observé, étant donné les intervalles de tolérance d'erreur calculés auparavant (compte*_{xy}*),
b) et à calculer la somme des intensités des masses d'ions fragmentaires observées coïncidant avec les masses d'ions fragmentaires théoriques (somme*_{xy}*).

7. Procédé conforme à la revendication 6, qui comporte en outre le fait de modéliser la distribution du nombre d'ions fragmentaires coïncidents selon un modèle de Poisson, et le fait de générer, pour chaque position, une valeur p pour la probabilité d'un accord entre un spectre d'ions fragmentaires observé et un spectre ou une partie de spectre d'ions théorique, et en option, le fait de corréler la distribution locale de cotes pour avoir un surplus de confiance, étant entendu que les positions où la valeur p est plus petite qu'un niveau prédéfini de signification sont considérées comme étant statistiquement significatives.

8. Procédé conforme à la revendication 6 ou 7, qui comporte en outre le fait d'annoter le spectre de fragments observé pour indiquer quels pics étaient accordés avec les ions fragmentaires théoriques, et le fait de mettre à jour la séquence pour indiquer quelle sous-séquence comportait des ions fragmentaires observés accordés.

9. Procédé conforme à l'une des revendications 1 à 8, dans lequel on ajuste les masses théoriques des ions fragmentaires en supposant un état de charge z : les « masses théoriques d'ion fragmentaire » sont mises à jour en « (masses théoriques de fragment)/z ».

10. Procédé conforme à l'une des revendications 1 à 9, dans lequel l'échantillon comprend plus d'une macromolécule biologique dudit organisme.

11. Système de traitement de données comprenant des moyens configurés pour mettre en oeuvre un procédé conforme à l'une des revendications 1 à 10.

12. Produit de type programme d'ordinateur, comportant des instructions qui, lorsqu'elles sont exécutées par un dispositif ou système informatique, font que ce dispositif ou système informatique exécute un procédé conforme à l'une des revendications 1 à 10.
